# EUROPEAN PATENT SPECIFICATION

(11) **EP 2 053 919 B1**
(45) Date of publication and mention of the grant of the patent: **25.12.2013**
(21) Application number: 07814124.9
(22) Date of filing: 15.08.2007
(51) Int. Cl.: A61K 31/137, A61P 25/00

(54) **METHOD OF REDUCING NEURONAL CELL DAMAGE**
VERFAHREN ZUR REDUZIERUNG VON SCHÄDIGUNGEN DER NERVENZELLEN
PROCEDE DE REDUCTION D'UN DOMMAGE AUX CELLULES NEURONALES

(30) Priority: 23.08.2006 US 839974 P
(43) Date of publication of application: 06.05.2009
(73) Proprietor: THE UNIVERSITY OF MONTANA, Missoula, MT 59812-4104 (US)
(72) Inventor: POULSEN, David, J., Missoula, MT 59803 (US); RAU, Thomas, Frederick, Stevensville, MT 59870 (US)
(74) Representative: Potter Clarkson LLP
(86) International application number: PCT/US2007/076034
(87) International publication number: WO 2008/024660

(56) References cited:
- MARTINSSON LOUISE ET AL: "Drugs for stroke recovery: the example of amphetamines.", DRUGS & AGING 2004 LNKD- PUBMED:14960125, vol. 21, no. 2, 2004, pages 67-79, XP009164481, ISSN: 1170-229X
- WANG Y ET AL: "Methamphetamine potentiates ischemia/reperfusion insults after transient middle cerebral artery ligation.", STROKE; A JOURNAL OF CEREBRAL CIRCULATION MAR 2001 LNKD- PUBMED:11239201, vol. 32, no. 3, March 2001 (2001-03), pages 775-782, XP002686833, ISSN: 1524-4628
- MATSUZAKI HIDEO ET AL: "Brain-derived neurotrophic factor rescues neuronal death induced by methamphetamine.", BIOLOGICAL PSYCHIATRY 1 JAN 2004 LNKD- PUBMED:14706425, vol. 55, no. 1, 1 January 2004 (2004-01-01), pages 52-60, XP002686834, ISSN: 0006-3223
- LUDOLPH A.G. ET AL.: 'Methylphenidate exerts no neurotoxic, but neuroprotective effects in vitro' J. NEURAL. TRANSM. vol. 113, 2006, pages 1927 - 1934, XP019461719
- Eakin (Thesis, Virginia Commonwealth Univ, May 2006), (see Abstract) XP008108886
- XU ET AL. NEUROPSYCHOPHARM. vol. 28, 2003, pages 53 - 62, XP008103968
- KUMAGE ET AL. JAPAN J. PHARMACOL. vol. 54, 1990, pages 407 - 413, XP008103967
- ARAKI ET AL. PHYSIOLOGY & BEHAVIOR vol. 74, 2001, pages 127 - 131, XP008103965
- BHARDWAJ ET AL. CURR. CARDIOL. RESP. March 2003, pages 167 - 167, XP008103958

## Description

### TECHNICAL FIELD

The present invention is directed to uses for reducing the occurrence of neuronal cell damage, including cell death, caused by transient cerebral hypoxia and/or ischemia, comprising the steps of: diagnosing a subject having a transient cerebral hypoxic and/or ischemic condition and within 16 hours after onset of the condition, administering to the subject a neuroprotective amount of a methamphetamine. Also disclosed herein but not claimed are uses involving a pharmaceutical agent selected from the group consisting of: a central nervous system stimulant (CNSS), monoamine neurotransmitter, monoamine oxidase inhibitor (MAOI), tricyclic antidepressant (TCA), or a combination thereof, such as amphetamines, methylphenidate, methylenedioxymethamphetamine, or a combination thereof.

### BACKGROUND OF THE INVENTION

Strokes are the leading cause of disability among adults, with over 80% involving ischemic insult. To date, no preventative or neuroprotective therapy has proven to be efficacious in humans. Amphetamines are one of the most extensively studied and promising group of drugs used to facilitate stroke recovery after neuronal cell damage has occurred (see (Martinsson and Eksborg 2004)). In rats, a single dose of amphetamines (e.g., dexamphetamine) administered 24 hrs after sensorimotor cortex ablation promotes hemiplegic recovery (Feeney et al. 1982). This beneficial effect has been confirmed in a variety of different focal injury models and species (Sutton et al. 1989; Hovda and Fenney 1984; Hovda and Feeney 1985; Schmanke et al. 1996; Dietrich et al. 1990; Stroemer et al. 1998). In each of these studies ischemic injury was modeled by the permanent ligation/embolism of a vascular component, or cortical ablation.

A different type of ischemic injury involves the transient interruption and reperfusion of blood flow to the brain. The hippocampus is extremely sensitive to this type of ischemic insult. In humans and experimental rodent models, brief ischemic episodes can result in the selective and delayed death of neurons located in the hippocampus, especially the pyramidal cells of the CAI sector (Kirino 1982). This type of lesion impairs performance on cognitive tasks that involve spatial memory (Zola-Morgan et al. 1986; Squire and Zola-Morgan 1991). Although amphetamine administration is associated with improved behavioral recovery in models of focal ischemia or cortical ablation, the prior art reported that treatment with amphetamines does not reduce infarct volume and thus, is not a preventative or neuronal protectant. The prior art also suggest that amphetamines facilitate behavioral recovery after cortical injury by influencing brain plasticity (Gold et al. 1984) as well as resolution of diaschisis ((Hovda et al. 1987; Sutton et al. 2000). The prior art, however, further teaches that amphetamines do not improve recovery following certain types of injury including lesions in the substantia nigra (Mintz and Tomer 1986). The prior art also teaches that administration of amphetamines (e.g., methamphetamine; MA) prior to focal ischemia actually increases the infarct volume in cortical and striatal regions (Wang et al. 2001).

A need still exist for a treatment that prevents neuronal damage before it occurs and actually provides neuronal protection after the occurrence of a transient cerebral hypoxic and/or ischemic condition to minimize or prevent damage. Such a preventative method is disclosed herein, which provides a method of preventing or reducing damage to the cerebral neuronal cells before it occurs instead of trying to treat the damage after occurrence and promote recovery.

### SUMMARY OF THE INVENTION

The present invention is defined in the accompanying claims and directed to uses for reducing the occurrence of neuronal cell damage caused by transient cerebral hypoxia and/or ischemia, comprising the steps of: diagnosing a subject having a transient cerebral hypoxic and/or ischemic condition; and within 16 hours after onset of the condition, administering to the subject a neuroprotective amount of a methamphetamine. Also disclosed herein but not claimed are uses involving a pharmaceutical agent selected from the group consisting of: a central nervous system stimulant (CNSS), monoamine neurotransmitter, monoamine oxidase inhibitor (MAOI), tricyclic antidepressant (TCA), or a combination thereof, such as amphetamines, methylphenidate, methylenedioxymethamphetamine, or a combination thereof.

In one specific embodiment, the pharmaceutical agent is methamphetamine administered to the subject in unit dosage amounts of less than 5 mg/kg.

In other specific embodiments, the pharmaceutical agent is a combination of methamphetamine, methylphenidate, methylenedioxymethamphetamine, or a combination thereof and at least one additional agent selected from the group consisting of: a monoamine neurotransmitter, MAOI, or a TCA. The additional agent can also include a monoamine neurotransmitter, preferably selected from the group consisting of: dopamine, norepinephrine, or serotonin.

The present invention reduces the occurrence of cerebral neuronal cell damage, which includes cell death, and more preferably, reduces the occurrence of neuronal cell damage to the neuronal cells. In a preferred embodiment, the present invention reduces the occurrence of neuronal cell damage to the neuronal cells of the hippocampus.

Typically, the transient cerebral hypoxic and/or ischemic condition is caused by loss of blood, a heart attack, strangulation, surgery (e.g., cardiac surgery), a stroke, air-way blockage, ischemic optic neuropathy, spinal cord injuries, traumatic brain injury, or low blood pressure. The condition, however, can be caused by many conditions, conditions that cause neuronal cell damage due to the lack of oxygen and/or glucose reaching the neuronal cells for a temporary period of time.

In certain preferred embodiments, the pharmaceutical agent is administered within 14, 12, 10, 8, 6, 4, or 2 hours after the onset of the condition. The agent is preferably administered via a parenteral or oral route, but other routes are contemplated and can be used depending on the condition.

In one embodiment, the pharmaceutical agent is administered in a pharmaceutical composition comprising a pharmaceutically acceptable carrier. The pharmaceutical composition can be an immediate or extended release formulation depending on the condition and likelihood of reoccurrence.

### BRIEF DESCRIPTION OF THE DRAWINGS

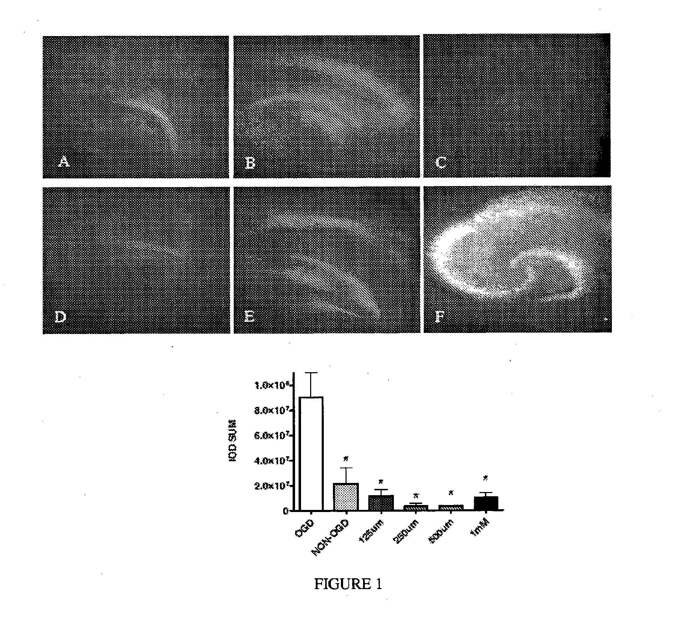

Figure 1: shows a neuroprotective dose response of MA following oxygen-glucose deprivation (OGD). Representative images of propidium iodide stained rat hippocampal slice cultures taken 48hrs post-OGD are shown. Cultures were treated with the following doses of MA: (A) Non-OGD control; (B) 125µm MA added 5min post-OGD; (C) 250µm MA added 5min post-OGD; (D) 500µm MA added 5min post-OGD; (E) 1mMµm MA added 5min post-OGD; (F) OGD only. Panal (G) shows statistical analysis of PI staining reported as relative fluorescence intensity (IOD). **=p<0.01, One-way ANOVA, Dunnet's Post-Hoc (OGD), error bars= mean & SEM; (OGD) n=10, (Non-OGD) n=13, (1mM MA) n=10, (500µM MA) n=11, (250µM MA) n=9, (125µM MA) n=7

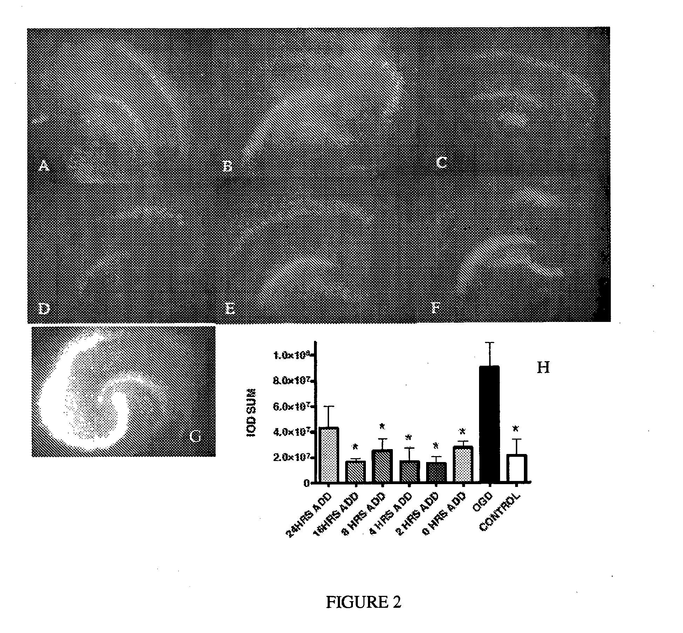

Figure 2: shows a temporal analysis of MA mediated neuroprotection following OGD. Representative images of propidium iodide stained rat hippocampal slice cultures taken 48hrs post-OGD are shown. A 250µM dose of MA was administered post-stroke at the time points indicated: (A) non-OGD; (B) 0-5min post-OGD; (C) 2hrs post-OGD; (D) 4hrs post-OGD; (E) 8hrs post-OGD; (F) 16hrs post-OGD; (G) OGD-untreated. Panal (H) shows statistical analysis of PI staining reported as relative fluorescence intensity (IOD). n=4, *=p<0.05, One-way ANOVA, Dunnet's post-hoc (OGD), error bars= mean & SEM

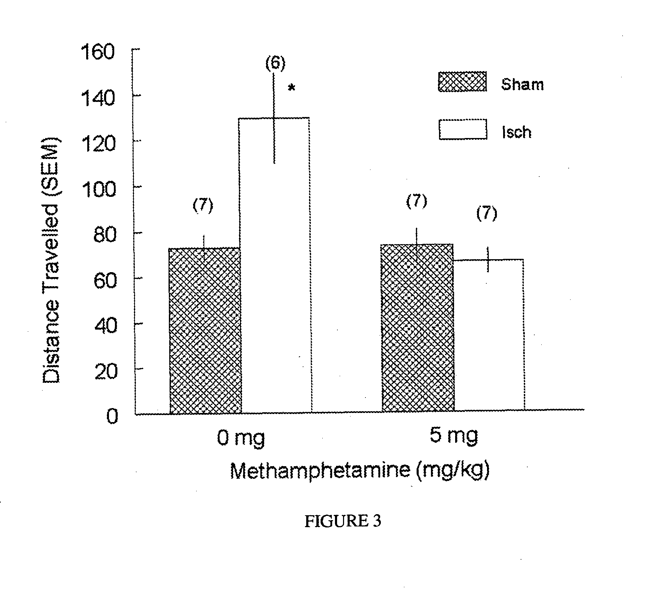

Figure 3: shows the Mean (±SEM) distance traveled in a novel open field apparatus. Animals were tested 24 hrs following 5-min 2-VO (Isch) or sham surgery (Sham). Following surgery (1-2 min), gerbils received methamphetamine (5 mg) or saline vehicle (0 mg). Gerbils were placed in the center region and permitted to explore the novel environment for 5 minutes and distance data were collected using an automated tracking system. Ischemic gerbils without methamphetamine treatment were significantly more active compared to the no drug sham group. Ischemic and sham gerbils treated with the drug were not different and drug treatment failed to significantly alter activity levels relative to the control condition. *P < 0.05 vs. Isch + drug condition.

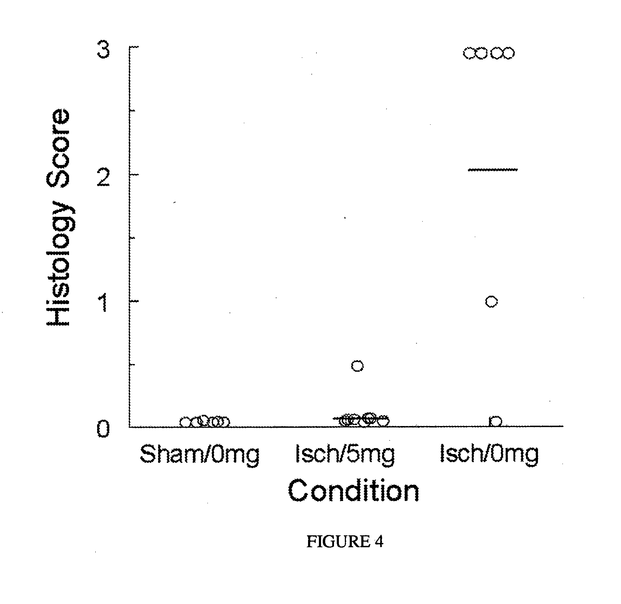

Figure 4: shows individual histological rating scores of hippocampal sections evaluated 21 days after ischemic insult (Isch) or sham control surgery (Sham). Gerbils were treated with methamphetamine (5 mg) or vehicle (0 mg) 1-2 minutes following surgery. Damage to the hippocampal CA1 region was evaluated using a 4 point rating scale. A score of 0 (4-5 compact layers of normal neuronal bodies), 1 (4-5 compact layers with presence of some altered neurons), 2 (spares neuronal bodies with "ghost spaces" and/or glial cells between them), 3 (complete absence or presence of only rare normal neuronal bodies with intense gliosis of the CA1 subfield) was assigned for each animal. Analysis revealed that treatment with methamphetamine significantly reduced damage to the hippocampal CA1 following ischemic insult.

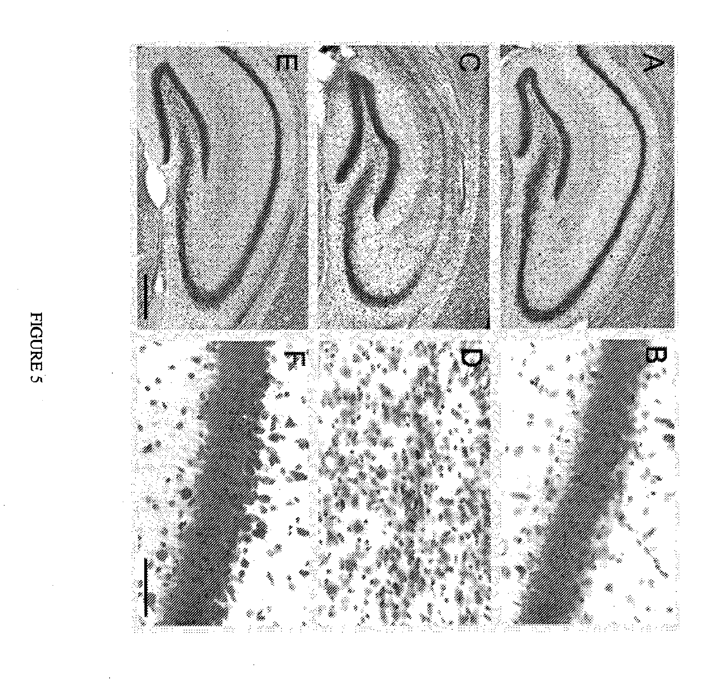

Figure 5: are photomicrographs of hippocampal sections processed 21 days after ischemic insult or sham procedure followed by administration of methamphetamine (5 mg/kg) or vehicle. A 5-min 2-VO resulted in the selective loss of pyramidal neurons in the hippocampal CA1 region (Panels C, D). As expected, sham surgery (Panels A, B) did not result in any neuronal cell loss. Gerbils treated with methamphetamine 1-2 minutes following ischemic insult failed to exhibit any damage to the hippocampus (Panels E, F). Sections were stained with cresyl violet. Scale bars = 200 µm (A, C, E) and 60 µm (B, D, F).

### DETAILED DESCRIPTION OF THE PREFERRED EMBODIMENTS

The present invention is defined in the accompanying claims and reduces the occurrence of cerebral neuronal cell damage, including cell death, caused by a transient cerebral hypoxic and/or ischemic condition. Preferably the invention reduces the occurrence of neuronal cell damage to the cells of the hippocampus. The transient cerebral hypoxic and/or ischemic condition can be caused by many conditions that cause lack of oxygen and/or glucose to the cerebral cells for a temporary period of time. For example, a heart attack, strangulation, surgery (e.g., cardiac surgery), a stroke, blood loss, air-way blockage, or low blood pressure. Preferably, the subject being treated is a mammal, e.g., monkey, dog, cat, horse, cow, sheep, pig, and more preferably the subject is a human.

In contrast to the prior art, the present invention actually provides protection and prevents damage to cerebral neuronal cells after the occurrence of transient cerebral hypoxia and/or ischemia instead of simply promoting recovery after the neuronal cell damage has already been caused. To provide the greatest neuronal protection to the subject, the neuroprotective agent should be administered to the subject within 16 hours after onset (e.g., 10, 8, 6, 4, 2 hours) of the transient cerebral hypoxic and/or ischemic condition. The neuroprotective agent is a methamphetamine. Other neuroprotective agents include: a central nervous system stimulant (CNSS), monoamine neurotransmitter, monoamine oxidase inhibitor (MAOI), tricyclic antidepressant (TCA), or a combination thereof.

Also disclosed herein but not claimed are uses involving amphetamine, methylphenidate, ethylenedioxymethamphetamine, or combinations thereof. Amphetamine is a compound containing a phenylethylamine. Phenylethylamine is a d-amphetamine, such as dextroamphetamine, for example, dextroamphetamine aspartate, dextroamphetamine sulfate, dextroamphetamine saccharate, etc. Specific examples include, ADREX, BIPHETAMINE, DESOXYN, DEXEDRINE, FERNDEX, ROBESE, SPANSULE, OXYDESS II, DEXTROSTAT.

In one embodiment, the pharmaceutical agent is administered in a pharmaceutical composition comprising a pharmaceutically acceptable carrier. The pharmaceutical composition can be an immediate or extended release formulation depending on the condition and likelihood of reoccurrence. The compositions can further include other pharmaceutically active compounds including, for example, at least one additional agent selected from the group consisting of: a monoamine neurotransmitter, MAOI, or a TCA. The additional agent can also include a monoamine neurotransmitter, preferably selected from the group consisting of: dopamine, norepinephrine, or serotonin, and more preferably norepinephrine.

Those skilled in the art will recognize various synthetic methodologies that may be employed to prepare non-toxic pharmaceutically acceptable compositions comprising the neuroprotective agent.

Pharmaceutical compositions can be prepared in individual dosage forms. Consequently, pharmaceutical compositions and dosage forms comprise the active ingredients as defined in the accompanying claims. The notation of "the pharmaceutical agent" or "neuroprotective agent" signifies the compounds as defined in the accompanying claims or salts thereof. Pharmaceutical compositions and dosage forms can further comprise a pharmaceutically acceptable carrier. In one embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which an active ingredient is administered. Such pharmaceutical carriers can be liquids, such as water and oils, including those of petroleum, animal, vegetable or synthetic origin, such as peanut oil, soybean oil, mineral oil, sesame oil and the like. The pharmaceutical carriers can be saline, gum acacia, gelatin, starch paste, talc, keratin, colloidal silica, urea, and the like. In addition, other excipients can be used.

Single unit dosage forms are suitable for oral, mucosal (e.g., nasal, sublingual, vaginal, buccal, or rectal), parenteral (e.g., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient. The agent is preferably administered via a parenteral or oral route, but other routes are contemplated as discussed in detail herein and largely depend on the ischemic condition.

The composition, shape, and type of dosage forms will typically vary depending on their route of administration and animal being treated. For example, a parenteral dosage form may contain smaller amounts of one or more of the active ingredients it comprises than an oral dosage form used to treat the same disease. These and other ways in which specific dosage forms will vary from one another will be readily apparent to those skilled in the art. See, e.g., Remington's Pharmaceutical Sciences, 18th ed., Mack Publishing, Easton Pa. (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water.

The pharmaceutical compositions and dosage forms may comprise one or more compounds that reduce the rate by which an active ingredient will decompose. Such compounds, which are referred to herein as "stabilizers," include, but are not limited to, antioxidants such as ascorbic acid, pH buffers, or salt buffers.

For a particular condition or method of treatment, the dosage is determined empirically, using known methods, and will depend upon facts such as the biological activity of the particular compound employed, the means of administrations, the age, health and body weight of the host; the nature and extent of the symptoms; the frequency of treatment; the administration of other therapies and the effect desired. Hereinafter are described various possible dosages and methods of administration with the understanding that the following are intended to be illustrative only. The actual dosages and method of administration or delivery may be determined by one of skill in the art. When the neuroprotective agent is methamphetamine administered to humans, the unit dosage amount is typically less than 5 mg/kg. Great dosages are generally toxic and should not typically be used.

Frequency of dosage may also vary depending on the compound used and whether an extended release formulation is used. However, for treatment of most disorders, a single dose is preferred.

### Oral Dosage Forms

Pharmaceutical compositions that are suitable for oral administration can be presented as discrete dosage forms, such as, but are not limited to, tablets (e.g., chewable tablets), caplets, capsules, and liquids (e.g., flavored syrups). Such dosage forms contain predetermined amounts of active ingredients, and may be prepared by methods of pharmacy well known to those skilled in the art. See generally, Remington's Pharmaceutical Sciences, 18th ed., Mack Publishing, Easton Pa. (1990).

Typical oral dosage forms are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (e.g., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, Natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, Pa.), and mixtures thereof. A specific binder is a mixture of microcrystalline cellulose and sodium carboxymethyl cellulose sold as AVICEL RC-581. Suitable anhydrous or low moisture excipients or additives include AVICEL-PH-103 and Starch 1500 LM.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions is typically present in from about 50 to about 99 weight percent of the pharmaceutical composition or dosage form.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, Md.), a coagulated aerosol of synthetic silica (marketed by Degussa Co. of Plano, Tex.), CAB-O-SIL (a pyrogenic silicon dioxide product sold by Cabot Co. of Boston, Mass.), and mixtures thereof. If used at all, lubricants are typically used in an amount of less than about 1 weight percent of the pharmaceutical compositions or dosage forms into which they are incorporated.

A preferred solid oral dosage form comprises an active ingredient, anhydrous lactose, microcrystalline cellulose, polyvinylpyrrolidone, stearic acid, colloidal anhydrous silica, and gelatin.

### Parenteral Dosage Forms

Parenteral dosage forms can be administered to patients by various routes including, but not limited to, subcutaneous, intravenous, bolus injection, intramuscular, and intraarterial. Because their administration typically bypasses patients' Natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### EXAMPLES

The neuroprotective efficacy of amphetamines following transient cerebral ischemic insult was not previously investigated. In the present study, methamphetamine (MA) was evaluated using *in vitro* and *in vivo* models of transient cerebral ischemia. For the *in vitro* model, rat hippocampal slice cultures were challenged with oxygen-glucose deprivation. In a second series of experiments, a 5-min 2-VO occlusion gerbil model was used in combination with behavioral testing to test the neuroprotective efficacy of MA *in vivo.* During the present study it was surprisingly discovered and demonstrated that MA administration within 16 hours following transient cerebral ischemia is actually neuroprotective, reducing neuronal cell damage, including death.

### Materials and methods

### 1.1 Animals

All experimental animal procedures were approved by the University Institutional Animal Care and Use Committee. Twenty-eight adult male Mongolian gerbils (Meriones unguiculatus) weighing 60-80 gm were used for the *in vivo* experiments. These animals were housed individually in a light- (12 h light/dark cycle) and temperature- (23°C) controlled environment. Commercial rodent pellets and water were provided ad libitum.

### 1.2 In vitro hippocampal slice studies

Neonatal rats (Sprague-Dawley) at postnatal Day 7 (P7) were decapitated and the hippocampi dissected out under sterile conditions. The hippocampi were chopped into 400µm slices on a Mcllwain tissue chopper and individual slices were cultured on Millicell permeable membranes (0.4µM pore size) in six well plates for 6 days at 37°C in 5% CO2. For the first two days, the slices were maintained in a primary plating media (50% DMEM (+) glucose, 25% HBSS (+) glucose, 25% heat inactivated horse serum, 5 mg/mL D-glucose (Sigma), ImM Glutamax, 1.5% PenStrep/Fungizone (Gibco), and 5 mL of 50X B27 (Gibco) supplement plus anti-oxidants that was changed every 24h. On the fourth day, the slices were placed in serum-free neurobasal medium (10mL Neurobasal-A, 200 µL of 50X B27 supplement, 100µL of 100X Fungizone, and 100 µL of 100X Glutamax) and this media was changed every 48hrs. 24hrs prior to experimentation, the inserts were placed in a serum-free neurobasal media and B27 supplement without antioxidants. Prior to the oxygen-glucose deprivation (OGD), a glucose free balanced salt solution (BSS) (120 mM NACl, 5mM KCl, 1.25 mM NaH2PO4, 2mM MgSO4, 2mM CaC12, 25 mM NaHCO3, 20 mM HEPES, 25 mM sucrose pH of 7.3) was infused for 1 hour with 5% C02 and 10L/hr nitrogen gas. The inserts were then transferred into deoxygenated BSS and placed in a 37o tank (Pro-Ox) with an oxygen feedback sensor that maintained gas levels at 0.1% 02, 5% C02, 94.4% Nitrogen for 90m. After OGD, the slices were immediately transferred back into prewarmed neurobasal media and assayed per experimental protocols.

### 1.3 Transient Cerebral ischemia

Gerbils were anesthetized with isoflurane and core-body temperature maintained at 37-38°C during surgery using a homeothermic blanket (Harvard Apparatus, South Natick, USA). A midline incision was made in the neck and the common carotid arteries were isolated and occluded for 5 min using 85-gm pressure aneurysm clips (ISCH; n=14). A second group of gerbils (SHAM; n= 14) underwent the identical procedure except the carotid arteries were not clamped. The incision was sutured and animals received MA (5 mg/ kg; i.p) or equal volume of vehicle (saline; 0 mg) within 2 minutes of reperfusion. Animals were placed in a warmed cage, and observed for 30 minutes. Tylenol (8 mg/ml) was added to drinking water to provide postoperative analgesia.

### 1.4 Behavioral Testing and Histological Evaluation.

Each gerbil was tested 48 hrs following surgery in an open-field apparatus consisting of a metal screen floor 77 cm X 77 cm with walls 15 cm in height. Animals were placed in the center region and permitted to explore the novel environment for 5 minutes. Behavioral data (distance traveled, speed) were collected using an automated tracking system (ANY-maze, Stoelting, IL) and evaluated separately using ANOVA and the appropriate post hoc test (P < 0.05 considered significant). Twenty-one days post-surgery, gerbils were euthanized with C02 and perfused with phosphate buffered saline followed by 4% paraformaldehyde. Tissue from sham gerbils treated with MA (SHAM + 0 mg) was not evaluated since acute administration of MA was not expected to histologically alter the hippocampus of this group. Brains were removed and post-fixed for at least 48 hrs prior to collection of 40 µm vibratome sections through the hippocampal region. Sections were mounted on slides and stained with cresyl violet. Damage to the hippocampal CA1 region was evaluated without knowledge of treatment condition by two independent observers using a 4 point rating scale described elsewhere (Babcock *et al.* 1993). A score of 0 (4-5 compact layers of normal neuronal bodies), 1 (4-5 compact layers with presence of some altered neurons), 2 (spares neuronal bodies with "ghost spaces" and/or glial cells between them), 3 (complete absence or presence of only rare normal neuronal bodies with intense gliosis of the CA1 subfield) was assigned for each animal. Ratings were averaged and evaluated using nonparametric statistics (Kruskal-Wallis and Mann-Whitney U test; P < 0.05 considered significant).

### Results

### 2.1 In vitro hippocampal slice studies

Hippocampal rat slices exposed to 90min of oxygen glucose deprivation (OGD) and treated with methamphetamine (MA) showed significantly (p=<0.01) decreased levels of propidium iodide (PI) uptake indicating decreased neuronal death when compared to OGD only slices (Figure 1). In dose response studies with MA, we observed optimal dosing with 250µM MA and increasing PI uptake as the concentration increased or decreased from this amount. However, at all concentrations tested (125 µM, 250 µM, 500 µM, 1mM) we observed significant neuroprotection (p=<0.01) when compared to OGD-only slices.

To further elucidate the effect of MA we added 250µM at various time points after OGD and found that MA significantly (p=<0.05) decreased neuronal death when administered up to 16hrs after OGD. Addition of MA 24hrs post-OGD decreased neuronal death but did not significantly differ from OGD.

### 2.2 Transient cerebral ischemia studies

Gerbils exhibited coordinated movements within 10 minutes of isoflorane termination. Animals treated with MA became piloerect with their tails pointing up. Animals were tested in an open field apparatus 48 hrs following surgery. Gerbils that underwent ischemic insult without MA treatment traveled 129.4 m (± 20; SEM), while sham controls with and without drug treatment traveled 72.7 m (± 6) and 73.2 m (± 7.5), respectively. Ischemic gerbils treated with MA following surgery traveled 66.3 m ± 5.6. Analysis of activity data revealed a significant interaction between drug treatment and surgical conditions (P < 0.05). Subsequent planned comparisons indicated that ischemic gerbils, in the absence of MA treatment, were significantly more active compared to the no drug sham group (P < 0.05). Ischemic and sham gerbils treated with MA were not significantly different (P > 0.05). Finally, treatment with MA failed to significantly alter activity levels relative to the control condition (SHAM + 0 mg vs. SHAM + 5 mg; P > 0.05). Analysis of speed data (distance traveled/time) revealed a similar pattern with ischemic gerbils treated with saline (ISCH exhibiting significantly fastest speeds relative to all other experimental groups (data not shown).

The histopathology scores and representative photomicrographs of the evaluated groups are illustrated in Figures 4 and 5, respectively. Gerbils in the ISCH + 0 mg condition exhibited extensive damage to the hippocampal CA1 region. Four of six gerbils in this group had complete absence of normal neuronal bodies with intense gliosis of the CA1 subfield. In contrast, all of the gerbils in the SHAM + 0 mg group were rated as having no detectable damage to the hippocampus (mean rating 0 ± 0). Six of the animals in the ISCH + 5 mg MA group exhibited 4-5 compact layers of normal neuronal bodies in the hippocampus (group rating .07 ± .07). Only 1 gerbil in this condition exhibited any detectable damage to the CA1 region. Analysis of rating scores revealed a significant difference between groups (P < 0.05). Subsequent evaluation of individual group data indicated that SHAM + 0 mg and ISCH + 5 mg conditions were not significantly different (P > 0.05) and both of these conditions were significantly different from the ISCH + 0 mg group (P < 0.05).

### Discussion

The results of the present study indicate that if a neuroprotective agent, e.g., MA, is administered within 16 hours after transient ischemic insult, damage to the neuronal cells may be reduced or prevented in the hippocampus. MA, for example, resulted in a dose-dependent neuroprotective response in rat hippocampal slice cultures challenged with oxygen-glucose deprivation. The 250µM dose showed the greatest degree of protection and was effective when administered up to 16 hours following oxygen-glucose deprivation. At 24hrs post-OGD MA administration did not significantly reduce PI uptake indicating that MA dosing must occur within a relatively short time period after OGD to activate the mechanism(s) responsible for reducing neuronal damage and death.

The neuroprotective efficacy of MA was also demonstrated *in vivo* using a 5-min gerbil 2-VO transient ischemia model. MA administration within 1-2 minutes of reperfusion prevented any significant loss of hippocampal CA1 pyramidal cells. The histological evaluation revealed that ischemic gerbils treated with MA exhibiting almost complete protection of the hippocampal CA1 region with only 1 of 7 animals exhibited any detectable neuronal pathology in the hippocampus. A 5-min bilateral carotid occlusion in the gerbil produces increased locomotor activity that correlates with hippocampal CA1 cell death (Wang and Corbett 1990; Babcock *et al.* 1993). The locomotor activity of ischemic gerbils treated with MA in the present study was comparable to control levels, which is indicative of significant neuroprotection. It is entirely possible that the arousal and hyperactivity that amphetamines produce could interact with the behavioral effects of ischemia. However, behavioral testing in the present study was conducted after the drug should have been metabolized (48 hrs). Consistent with this interpretation was the observation that control gerbils treated with MA were not hyperactive relative to animals that received saline (SHAM + 0 mg). The dose of MA used in the *in vivo* experiment was derived from a previous report that used gerbils (Teuchert-Noodt *et al.* 2000; Araki *et al.* 2002) as an experimental model. We also conducted a preliminary study in which doses of MA greater than 5 mg/kg (*e.g*., 10 and 20 mg/kg) were found to be lethal in gerbils following surgery and were not evaluated further.

Amphetamine in combination with training has been shown to be a promising pharmacological strategies for behavioral recovery from stroke (see Martinsson and Eksborg, 2004). Our observation that MA actually prevents detectable hippocampal damage following ischemic insult if given within a particular time frame after insult, i.e., within 16 hours, represents a novel finding. It is notable that these findings show that neuroprotection is independent of any behavioral training following the insult. It is possible that the ability of MA to actually protect and prevent the hippocampus from neuronal damage, in contrast to the prior art teachings of treatment after damage has occurred, is effect in with transient cerebral ischemia. Unlike focal ischemia or other types of cortical injury, transient cerebral ischemia is characterized by a pattern of delayed cell death limited to hippocampal pyramidal cells. The reperfusion that follows the brief ischemic episode in this model is a key event for the subsequent cell death that occurs 3-5 days following insult.

Current studies of MA administration prior to an acute stroke event indicate that MA significantly increases neuronal death (Wang et a/. 2001). However, in light of our current findings, it is entirely possible that treatment with MA prior to a stroke event depletes stores of dopamine and norepinephrine that remain unavailable for release after a stroke event, and the subsequent decrease in neuronal signaling may be playing a key role in the damage observed in MA pre-treatment and stroke. The ability of CNSS, e.g., MA, to induce an extremely large release of these neurotransmitters in a very short time span may partially explain the neuroprotective effect we observed in our experiments. Future research aimed at understanding the neuroprotective mechanism of CNSS agents may further elucidate the exact mechanism and treatment for acute ischemic events.

### References

Araki, H., Yamamoto, T., Kobayashi, Y., Futagami, K., Kawasaki, H., Gomita, Y. 2002. Effect of methamphetamine and imipramine on cerebral ischemia- induced hyperactivity in Mongolian gerbils. Japan Journal of Pharmacology 88, 293-299.
Babcock, A.M., Baker, D. A., Lovac, R. 1993. Locomotor activity in the ischemic gerbil. Brain Research 625, 351-354.
Boyeson, M.G., Feeney, D.M. 1990. Intraventricular norepinephrine facilitates motor recovery following sensorimotor cortex injury. Pharmacology Biochemistry Behavior 35, 497-501.
Boyeson, M.G., Harmon, R. L., Jones, J. L. 1994. Comparative effects of fluoxetine, amitriptyline and serotonin on functional motor recovery after sensorimotor cortex injury. American Journal Physical Medicine Rehabilitation 73, 76-83.
Culmsee C, Semkova L, Krieglstein J. 1999. NGF mediates the neuroprotective effect of the beta2-adrenoceptor agonist clenbuterol in vitro and in vivo: evidence from an NGF- antisense study. Neurochemistry International 35, 47-57.
Dietrich W.D., Alonso O., Busto R., Watson B.D., Loor Y., Ginsberg M.D. 1990. Influence of amphetamine treatment on somatosensory function of the normal and infarcted rat brain. Stroke 21, III147-III150.
Feeney D.M., Gonzalez A., Law W.A. 1982. Amphetamine, haloperidol, and experience interact to affect rate of recovery after motor cortex injury. Science 217, 855-857.
Follesa P., Mocchetti I. 1993. Regulation of basic fibroblast growth factor and nerve growth factor mRNA by beta-adrenergic receptor activation and adrenal steroids in rat central nervous system. Molecular Pharmacology 43, 132-138.
Gold P.E., Delanoy R.L., Merrin J. 1984. Modulation of long-term potentiation by peripherally administered amphetamine and epinephrine. Brain Research 305, 103-107.
Hovda D.A., Feeney D.M. 1985. Haloperidol blocks amphetamine induced recovery of binocular depth perception after bilateral visual cortex ablation in cat. Proceedings Western Pharmacology Society 28, 209-211.
Hovda D.A., Fenney D.M. 1984. Amphetamine with experience promotes recovery of locomotor function after unilateral frontal cortex injury in the cat. Brain Research 298, 358-361.
Hovda D.A., Sutton R.L., Feeney D.M. 1987. Recovery of tactile placing after visual cortex ablation in cat: a behavioral and metabolic study of diaschisis. Experimental Neurology 97, 391-402.
Kirino T. 1982. Delayed neuronal death in the gerbil hippocampus following ischemia. Brain Research 239, 57-69.
Martinsson L., Eksborg S. 2004. Drugs for stroke recovery: the example of amphetamines. Drugs Aging 21, 67-79.
Mintz M., Tomer R. 1986. Exposure to amphetamine after substantia nigra lesion interferes with the process of behavioral recovery. Pharmacology Biochemistry Behavior 25, 1307-1311:
Schmanke T.D., Avery R.A., Barth T.M. 1996. The effects of amphetamine on recovery of function after cortical damage in the rat depend on the behavioral requirements of the task. Journal of Neurotrauma 13, 293-307.
Semkova I., Schilling M., Henrich-Noack P., Rami A., Krieglstein J. 1996. Clenbuterol protects mouse cerebral cortex and rat hippocampus from ischemic damage and attenuates glutamate neurotoxicity in cultured hippocampal neurons by induction of NGF. Brain Research 717, 44-54.
Squire L.R., Zola-Morgan S. 1991. The medial temporal lobe memory system. Science 253, 1380-1386.
Stroemer R.P., Kent T.A., Hulsebosch C.E. 1998. Enhanced neocortical neural sprouting, synaptogenesis, and behavioral recovery with D-amphetamine therapy after neocortical infarction in rats. Stroke 29, 2381-2393.
Sutton R.L., Hovda D.A., Chen M.J., Feeney D.M. 2000. Alleviation of brain injury-induced cerebral metabolic depression by amphetamine, a cytochrome oxidase histochemistry study. Neural Plasticity 7, 109-125.
Sutton R.L., Hovda D.A., Feeney D.M. 1989. Amphetamine accelerates recovery of locomotor function following bilateral frontal cortex ablation in cats. Behavioral Neuroscience 103, 837-841.
Teuchert-Noodt G, Dawirs RR, Hildebrandt K. 2000. Adult treatment with methamphetamine transiently decreases dentate granule cell proliferation in the gerbil hippocampus. Journal Neural Transmission 107, 133-143.
Wang D., Corbett D. 1990. Cerebral ischemia, locomotor activity and spatial mapping. Brain Research 533, 78-82.
Wang Y., Hayashi T., Chang C.F., Chiang Y.H., Tsao L.I., Su T.P., Borlongan C., Lin S.Z. 2001. Methamphetamine potentiates ischemia/reperfusion insults after transient middle cerebral artery ligation. Stroke 32, 775-782.
Zola-Morgan S., Squire L.R., Amaral D.G. 1986. Human amnesia and the medial temporal region: Enduring memory impairment following a bilateral lesion limited to field CA1 of the hippocampus. Journal of Neuroscience 6, 2960-2967.

## Claims

1. Use of a methamphetamine in the manufacture of a medicament for reducing the occurrence of brain cell damage or death caused by transient cerebral hypoxia and/or ischemia in a subject, by a method comprising the steps of: identifying a subject having a transient cerebral hypoxic and/or ischemic condition; and within 16 hours after onset of the condition, administering to the subject a therapeutically effective amount of a methamphetamine.

2. A medicament comprising methamphetamine for use in reducing the occurrence of brain cell damage or death caused by transient cerebral hypoxia and/or ischemia in a subject, by a method comprising the steps of: identifying a subject having a transient cerebral hypoxic and/or ischemic condition; and within 16 hours after onset of the condition, administering to the subject a therapeutically effective amount of a methamphetamine.

3. The use of Claim 1 or the medicament for use according to Claim 2, wherein the methamphetamine administered to the subject in unit dosage amounts of less than 5 mg/kg.

4. The use of Claim 1 or the medicament for use according to Claim 2, wherein treatment reduces the occurrence of neuronal cell damage to brain cells of the hippocampus.

5. The use of Claim 1 or the medicament for use according to Claim 2, wherein the condition is caused by low blood pressure, blood loss, a heart attack, traumatic brain injury, strangulation, surgery, a stroke, ischemic optic neuropathy, or air-way blockage.

6. The use or medicament for use according to Claim 5, wherein the condition is caused by cardiac surgery.

7. The use or medicament for use according to Claim 5, wherein the condition is caused by traumatic brain injury.

8. The use of Claim 1 or the medicament for use according to Claim 2, wherein administration occurs within 14 hours after onset of the condition and only a single dose of the methamphetamine is administered.

9. The use of Claim 1 or the medicament for use according to Claim 2, wherein the administering is via a bolus injection.

10. The use or medicament for use according to Claim 9, wherein the methamphetamine is in a pharmaceutical composition comprising a pharmaceutically acceptable carrier.

11. The use or medicament for use according to Claim 10, wherein the pharmaceutical composition is an extended release formulation.

12. The use of Claim 1 or the medicament for use according to Claim 2, wherein the medicament consists of methamphetamine and a pharmaceutically acceptable carrier.

13. The use of Claim 1 or the medicament for use according to Claim 2, wherein the subject is a human in need of such treatment.

14. The use or medicament for use according to Claim 13, wherein the condition is caused by an ischemic stroke, cardiac surgery or ischemic optic neuropathy.

15. The use or medicament for use according to Claim 13, wherein the methamphetamine is administered within 12 hours of surgery.

16. The use or medicament for use according to Claim 13, wherein the methamphetamine is administered within 2 hours of surgery.

17. Use of a methamphetamine in the manufacture of a medicament for reducing the occurrence of brain cell damage or death caused by traumatic brain injury in a subject, by a method comprising the steps of: identifying a subject having traumatic brain injury and within 16 hours after onset of the injury, administering to the subject a therapeutic effective amount of a methamphetamine.

18. A medicament comprising methamphetamine for use in reducing the occurrence of brain cell damage or death caused by traumatic brain injury in a subject, by a method comprising the steps of: identifying a subject having traumatic brain injury and within 16 hours after onset of the injury, administering to the subject a therapeutically effective amount of a methamphetamine.

19. The use of Claim 17 or the medicament for use according to Claim 18, wherein the medicament consists of methamphetamine and a pharmaceutically acceptable carrier.

## Patentansprüche

1. Verwendung eines Methamphetamins bei der Herstellung eines Medikaments zur Verringerung des Auftretens von Neuronenschädigung oder -tod, die durch eine transiente zerebrale Hypoxie und/oder Ischämie verursacht sind, bei einem Subjekt durch ein Verfahren mit den Stufen des Identifizierens eines Subjekts mit einem transienten zerebralen hypoxischen und/oder ischämischen Zustand und des Verabreichens einer therapeutisch wirksamen Menge eines Methamphetamins an das Subjekt innerhalb von 16 h nach dem Einsetzen des Zustands.

2. Methamphetamin umfassendes Medikament zur Verwendung bei der Verringerung des Auftretens von Neuronenschädigung oder - tod, die durch eine transiente zerebrale Hypoxie und/oder Ischämie verursacht sind, bei einem Subjekt durch ein Verfahren mit den Stufen des Identifizierens eines Subjekts mit einem transienten zerebralen hypoxischen und/oder ischämischen Zustand und des Verabreichens einer therapeutisch wirksamen Menge eines Methamphetamins an das Subjekt innerhalb von 16 h nach dem Einsetzen des Zustands.

3. Verwendung nach Anspruch 1 oder Medikament zur Verwendung nach Anspruch 2, wobei das Methamphetamin in Dosierungseinheitsmengen von weniger als 5 mg/kg an das Subjekt verabreicht wird.

4. Verwendung nach Anspruch 1 oder Medikament zur Verwendung nach Anspruch 2, wobei die Behandlung das Auftreten einer neuronalen Zellschädigung an Neuronen des Hippocampus verringert.

5. Verwendung nach Anspruch 1 oder Medikament zur Verwendung nach Anspruch 2, wobei der Zustand durch niedrigen Blutdruck, Blutverlust, eine Herzattacke, eine traumatische Hirnverletzung, Strangulation, einen chirurgischen Eingriff, einen Schlaganfall, eine ischämische Optikusneuropathie oder eine Luftwegblockade verursacht ist.

6. Verwendung oder Medikament zur Verwendung nach Anspruch 5, wobei der Zustand durch eine Herzoperation verursacht ist.

7. Verwendung oder Medikament zur Verwendung nach Anspruch 5, wobei der Zustand durch eine traumatische Hirnverletzung verursacht ist.

8. Verwendung nach Anspruch 1 oder Medikament zur Verwendung nach Anspruch 2, wobei die Verabreichung innerhalb von 14 h nach dem Einsetzen des Zustands erfolgt und nur eine Einzeldosis des Methamphetamins verabreicht wird.

9. Verwendung nach Anspruch 1 oder Medikament zur Verwendung nach Anspruch 2, wobei die Verabreichung durch eine Bolusinjektion erfolgt.

10. Verwendung oder Medikament zur Verwendung nach Anspruch 9, wobei das Methamphetamin in einer pharmazeutischen Zusammensetzung vorliegt, die einen pharmazeutisch akzeptablen Träger umfasst.

11. Verwendung oder Medikament zur Verwendung nach Anspruch 10, wobei die pharmazeutische Zusammensetzung eine Formulierung mit verlängerter Freisetzung ist.

12. Verwendung nach Anspruch 1 oder Medikament zur Verwendung nach Anspruch 2, wobei das Medikament aus Methamphetamin und einem pharmazeutisch akzeptablen Träger besteht.

13. Verwendung nach Anspruch 1 oder Medikament zur Verwendung nach Anspruch 2, wobei das Subjekt ein eine derartige Behandlung benötigender Mensch ist.

14. Verwendung oder Medikament zur Verwendung nach Anspruch 13, wobei der Zustand durch einen ischämischen Schlaganfall, eine Herzoperation oder ischämische Optikusneuropathie verursacht ist.

15. Verwendung oder Medikament zur Verwendung nach Anspruch 13, wobei das Methamphetamin innerhalb von 12 h nach einem chirurgischen Eingriff verabreicht wird.

16. Verwendung oder Medikament zur Verwendung nach Anspruch 13, wobei das Methamphetamin innerhalb von 2 h nach einem chirurgischen Eingriff verabreicht wird.

17. Verwendung eines Methamphetamins bei der Herstellung eines Medikaments zur Verringerung des Auftretens von Neuronenschädigung oder -tod, die durch eine traumatische Hirnverletzung verursacht sind, bei einem Subjekt durch ein Verfahren mit den Stufen des Identifizierens eines Subjekts mit einer traumatischen Hirnverletzung und des Verabreichens einer therapeutisch wirksamen Menge eines Methamphetamins an das Subjekt innerhalb von 16 h nach dem Einsetzen der Verletzung.

18. Methamphetamin umfassendes Medikament zur Verwendung bei der Verringerung des Auftretens von Neuronenschädigung oder - tod, die durch eine traumatische Hirnverletzung verursacht sind, bei einem Subjekt durch ein Verfahren mit den Stufen des Identifizierens eines Subjekts mit einer traumatischen Hirnverletzung und des Verabreichens einer therapeutisch wirksamen Menge eines Methamphetamins an das Subjekt innerhalb von 16 h nach dem Einsetzen der Verletzung.

19. Verwendung nach Anspruch 17 oder Medikament zur Verwendung nach Anspruch 18, wobei das Medikament aus Methamphetamin und einem pharmazeutisch akzeptablen Träger besteht.

## Revendications

1. Utilisation d'une méthamphétamine dans la fabrication d'un médicament destiné à réduire l'occurrence d'une lésion ou mort de cellules cérébrales provoquée par une hypoxie et/ou ischémie cérébrale(s) transitoire(s) transitoire(s) chez un sujet, par une méthode comprenant les étapes : d'identification d'un sujet ayant une affection hypoxique et/ou ischémique cérébrale transitoire ; et dans les 16 heures après le début de l'affection, d'administration au sujet d'une quantité thérapeutiquement efficace d'une méthamphétamine.

2. Médicament comprenant une méthamphétamine à utiliser dans la réduction de l'occurrence d'une lésion ou mort de cellules cérébrales provoquée par une hypoxie et/ou une ischémie cérébrale(s) transitoire(s) chez un sujet, par une méthode comprenant les étapes : d'identification d'un sujet ayant une affection hypoxique et/ou ischémique cérébrale transitoire ; et dans les 16 heures après le début de l'affection, d'administration au sujet d'une quantité thérapeutiquement efficace d'une méthamphétamine.

3. Utilisation selon la revendication 1, ou médicament à utiliser selon la revendication 2, dans lequel la méthamphétamine administrée au sujet en dosages unitaires s'élève à moins de 5 mg/kg.

4. Utilisation selon la revendication 1, ou médicament à utiliser selon la revendication 2, dans lequel le traitement réduit l'occurrence de la lésion de cellules neuronales à des cellules cérébrales de l'hippocampe.

5. Utilisation selon la revendication 1, ou médicament à utiliser selon la revendication 2, dans lequel l'affection est provoquée par une tension artérielle faible, une perte de sang, une attaque cardiaque, une lésion traumatique du cerveau, une strangulation, une intervention chirurgicale, un accident vasculaire cérébral, une neuropathie optique ischémique ou un blocage des voies aériennes.

6. Utilisation ou médicament à utiliser selon la revendication 5, dans lequel l'affection est provoquée par une chirurgie cardiaque.

7. Utilisation ou médicament à utiliser selon la revendication 5, dans lequel l'affection est provoquée par une lésion traumatique du cerveau.

8. Utilisation selon la revendication 1, ou médicament à utiliser selon la revendication 2, dans lequel l'administration se produit dans les 14 heures après le début de l'affection et seule une dose unique de la méthamphétamine est administrée.

9. Utilisation selon la revendication 1, ou médicament à utiliser selon la revendication 2, dans lequel l'administration se fait via une injection d'un bolus.

10. Utilisation ou médicament à utiliser selon la revendication 9, dans lequel la méthamphétamine est dans une composition pharmaceutique comprenant un véhicule pharmaceutiquement acceptable.

11. Utilisation ou médicament à utiliser selon la revendication 10, dans lequel la composition pharmaceutique est une formulation à libération prolongée.

12. Utilisation selon la revendication 1, ou médicament à utiliser selon la revendication 2, dans lequel le médicament consiste en de la méthamphétamine et un véhicule pharmaceutiquement acceptable.

13. Utilisation selon la revendication 1, ou médicament à utiliser selon la revendication 2, dans lequel le sujet est un être humain nécessitant un tel traitement.

14. Utilisation ou médicament à utiliser selon la revendication 13, dans lequel l'affection est provoquée par un accident vasculaire cérébral ischémique, une chirurgie cardiaque ou une neuropathie optique ischémique.

15. Utilisation ou médicament à utiliser selon la revendication 13, dans lequel la méthamphétamine est administrée dans les 12 heures après la chirurgie.

16. Utilisation ou médicament à utiliser selon la revendication 13, dans lequel la méthamphétamine est administrée dans les 2 heures après la chirurgie.

17. Utilisation d'une méthamphétamine dans la fabrication d'un médicament destiné à réduire l'occurrence d'une lésion ou mort de cellules cérébrales provoquée par une lésion traumatique du cerveau chez un sujet, par une méthode comprenant les étapes : d'identification d'un sujet ayant une lésion traumatique du cerveau et dans les 16 heures après le début de la lésion, d'administration au sujet d'une quantité thérapeutique efficace d'une méthamphétamine.

18. Médicament comprenant une méthamphétamine à utiliser dans la réduction de l'occurrence d'une lésion ou mort de cellules cérébrales provoquée par une lésion traumatique du cerveau chez un sujet, par une méthode comprenant les étapes : d'identification d'un sujet ayant une lésion traumatique du cerveau et dans les 16 heures après le début de la lésion, d'administration au sujet d'une quantité thérapeutique efficace d'une méthamphétamine.

19. Utilisation selon la revendication 17 ou médicament à utiliser selon la revendication 18, dans lequel le médicament consiste en de la méthamphétamine et un véhicule pharmaceutiquement acceptable.
